# EUROPEAN PATENT APPLICATION

(11) **EP 2 857 002 A1**
(43) Date of publication of application: **08.04.2015**
(21) Application number: 13796731.1
(22) Date of filing: 30.05.2013
(51) Int. Cl.: A61K 8/36, A61K 8/37, A61K 8/49, A61K 8/63, A61K 31/506, A61K 31/5575, A61K 31/568, A61K 47/10, A61P 17/14, A61Q 7/00

(54) **COMPOSITION FOR HAIR GROWTH**

(30) Priority: 30.05.2012 JP 2012123316
(71) Applicant: Asahi Glass Company, Limited, Tokyo 100-8405 (JP); Santen Pharmaceutical Co., Ltd, Osaka-shi, Osaka 530-8552 (JP)
(72) Inventor: SHIMAZAKI, Atsushi, Osaka-shi Osaka 533-8651 (JP); YASUDA, Arata, Tokyo 100-8405 (JP); MATSUMURA, Yasushi, Tokyo 100-8405 (JP)
(74) Representative: Müller-Boré & Partner Patentanwälte PartG mbB
(86) International application number: PCT/JP2013/065075
(87) International publication number: WO 2013/180229

(57) **Abstract**

To provide a composition for hair growth (hair growth agent) which has high hair growing effects and can be used for prevention or treatment of alopecia. The composition for hair growth comprises as an active ingredient at least one member selected from the group consisting of a compound represented by the formula (1) and a salt thereof. In the formula, R is a carboxy group or an alkoxycarbonyl group.

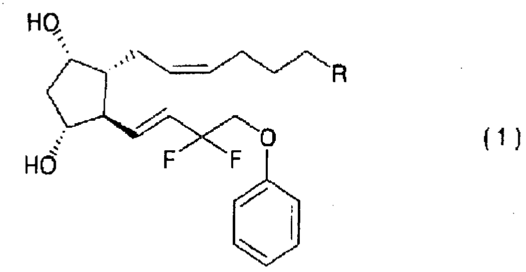

## Description

### TECHNICAL FIELD

The present invention relates to a composition for hair growth (hair growth agent) which comprises as an active ingredient a 15,15-difluoroprostaglandin F2α derivative. Such a composition for hair growth is useful as a preventive or treating agent for a disorder relating to hair such as alopecia and can be used also as a hair care product for the purpose of hair growth, hair restoration, hair increase, pilatory treatment, etc.

### BACKGROUND ART

Human hair is said to consist of from about 100,000 to 150,000 hairs. Such hairs repeat a cycle of anagen, catagen, telogen and defluxion for a period of 3- to 6-years. As a result, from 50 to 200 hairs on average per day are depleted. In such a cycle, if the ratio of hairs in anagen stage decreases and the ratio of hairs in telogen or catagen stage increases, symptoms of alopecia tend to appear.

Hairs are classified into downy hairs, vellus hairs, terminal hairs and modified terminal hairs depending upon the presence or absence of hair medulla and the presence or absence of melanin pigment.
(1) Downy hairs: Hairs which contain almost no hair medulla or melanin pigment, and many of them are depleted soon after birth. Their length is short, and the thickness is also slender at a level of from 20 to 30 µm. The hair surface is said to be rough as compared with other thick hairs, and no substantial difference is observed between the hair root and the hair shaft.
(2) Vellus hairs: Short, soft and very slender hairs which have no hair medulla and contain almost no melanin pigment and which are distributed over a wide portion of skin. Hair bulbs are located at the surface in the dermis.
(3) Terminal hairs: Hairs which have hair medulla and melanin pigment and usually occupy the majority of human hair and which can easily be observed directly by the naked eye. Hair bulbs of hair follicles are located deep in the dermis.
(4) Modified terminal hairs: Refer to eyebrows, eyelashes, beards, hircus, pubic hairs, etc. They have hair medulla and melanin pigment.

In alopecia, as the symptoms progress, a change of terminal hairs to vellus hairs will take place, whereby terminal hairs tend to change into a type of vellus hairs. Especially in the case of male pattern alopecia, the change of terminal hairs to vellus hairs is the most distinct change on appearance.

Further, another pattern relating to alopecia takes place due to a remarkable decrease of hair follicles. In the case of bald-head, it is said that hair follicles are likely to be atrophied so that about 1/3 thereof may be lost.

Alopecia is brought about by any one of the above-mentioned factors i.e. (1) an increase in the ratio of hairs in telogen or catagen stage, (2) a change of terminal hairs to vellus hairs and (3) a decrease in the number of hair follicles, or by a combination of such factors.

Such factors are considered to be brought about by one or a plurality of causes such as a decrease in the function of hair follicles due to male hormone, a decrease in the function of metabolism of hair follicles and hair roots, a decrease in the physiology of scalp, disturbances of blood flow in scalp, nutrient deficiency, stress, a side effect of medicine, advancing age, heredity, etc. and thus bring about alopecia. However, the real causes have not been clearly understood.

Heretofore, for the prevention or treatment of alopecia, it has been common to use one of, or a plurality in combination of, nutritional support (amino acids, vitamins, etc.) for hairs and hair follicles, blood circulation promotion by focal stimulation, strengthening of hair follicle function, a blood circulation promotor, an anti-androgen medicine, an antiseborrheic medicine, a keratolytic medicine, a bactericidal anti-inflammatory medicine, etc.

Recently, minoxidil, finasteride, etc. have been frequently used, since they are regarded as effective for the prevention or treatment of alopecia.

Also latanoprost as a prostaglandin F analog is known to have a hair growing function or a hair rearing function (Patent Documents 1 and 2).

On the other hand, a 15,15-difluoroprostaglandin F2α derivative is disclosed in Patent Documents 3 and 4, a fluorinated prostaglandin F2α derivative having a polysubstituted aryloxy group is disclosed in Patent Document 5, an ether-type difluoroprostaglandin F2α derivative is disclosed in Patent Document 6, and a difluoroprostaglandin F2α amide derivative is disclosed in Patent Document 7, respectively, as active ingredients for the prevention or treatment of eye diseases. Further, a retinal neuronal cell protective function of a 15,15-difluoroprostaglandin F2α derivative is disclosed in Patent Document 8. Still further, an eyelash growth-promoting function of a 15,15-difluoroprostaglandin F2α derivative is disclosed in Patent Document 9.

### PRIOR ART DOCUMENTS

### PATENT DOCUMENTS

Patent Document 1: WO2001/074315
Patent Document 2: WO98/33497
Patent Document 3: European Patent Application Publication No. 850926
Patent Document 4: JP-A-2004-002462
Patent Document 5: JP-A-10-259179
Patent Document 6: JP-A-2002-293771
Patent Document 7: JP-A-2003-321442
Patent Document 8: JP-A-2006-306862
Patent Document 9: WO2010/027040

### DISCLOSURE OF INVENTION

### TECHNICAL PROBLEM

However, particularly with respect to scalp hair growth promotion (including hair growth, hair volume increase, pilatory treatment, etc.), it is desired to have a more effective medicine developed. Further, if minoxidil is applied to e.g. the scalp, not only the hair growth promoting effect but also undesirable effects such as tanning of the skin, blackening of the skin, etc. are likely to be brought about.

Under the circumstances, it is an object of the present invention to solve such a problem and to provide a composition for hair growth (hair growth agent) having a high hair growing effect, which is useful for the prevention or treatment of alopecia particularly for scalp hair.

### SOLUTION TO PROBLEM

The present invention has the following constructions.
(1) A composition for hair growth, which comprises as an active ingredient at least one member selected from the group consisting of a compound represented by the formula (1) and a salt thereof: 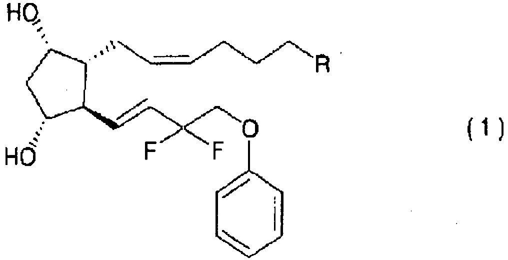 wherein R is a carboxy group or an alkoxycarbonyl group.
(2) The composition for hair growth according to the above (1), wherein in the compound represented by the formula (1), R is an isopropyloxycarbonyl group.
(3) The composition for hair growth according to the above (1) or (2), which further contains as an active ingredient at least one member selected from the group consisting of minoxidil and finasteride.
(4) The composition for hair growth according to any one of the above (1) to (3), which is used for prevention or treatment of male pattern alopecia.
(5) The composition for hair growth according to any one of the above (1) to (4), which is a liquid for external use comprising from 0.00001 to 0.01 w/v% of at least one member selected from the group consisting of a compound represented by the above formula (1) and a salt thereof, and from 40 to 95 v/v% of ethanol.
(6) The composition for hair growth according to the above (5), wherein the liquid for external use contains at least one polar solvent having a boiling point of at most 100°C.
(7) The composition for hair growth according to the above (6), wherein the polar solvent is ethanol, or a mixture of ethanol and water.
(8) The composition for hair growth according to any one of the above (5) to (7), which is a hair care product.
(9) A cosmetic method for hair, which comprises applying to hair the composition for hair growth as defined in any one of the above (1) to (8).

### ADVANTAGEOUS EFFECTS OF INVENTION

The above active ingredient is one having a strong hair growth promoting effect without bringing about blackening of the skin, and thus, according to the present invention, it is possible to obtain a high hair growth promoting effect without bringing about blackening of the skin.

Accordingly, the composition for hair growth of the present invention is useful as an agent for preventing or treating a disorder relating to hair such as alopecia, and can be used as various types of hair care products such as a hair growth agent, a hair restoration agent, a hair increase agent, a pilatory agent, etc.

### DESCRIPTION OF EMBODIMENTS

The present invention is a composition for hair growth which comprises as an active ingredient at least one member selected from 15,15-difluoroprostaglandin F2α derivatives and which is to be used for hair (human hair or animal body hair), particularly for scalp hair.

With respect to 15,15-difluoroprostaglandin F2α derivatives, the present inventors have found that a compound of the above formula (1) or a salt thereof has a hair growing effect for body hair, particularly for scalp hair, and brings about no blackening of the skin at the applied portion, and thus, they have succeeded to realize a hair growth agent of new type.

Further, the composition for hair growth of the present invention does not bring out blackening of the skin like minoxidil and is free from an undesirable effect such as blackening of the skin, and thus, it is not only highly useful as a hair care product, but also extremely useful for cosmetic purposes.

Heretofore, it is known that the effect of inducing an anagen stage of hair is detected by using, as an index, blackening of the skin which can be visually ascertained (e.g. JP-A-2003-149230). However, as in the present invention, the hair growth-promoting effect of a compound which brings about no blackening of the skin, is considered to be different from the working mechanism of the conventional active ingredient.

Especially, it has been confirmed that a 15,15-difluoroprostaglandin F2α derivative of the formula (1) wherein R is an isopropyloxycarbonyl group (Tafluprost), is capable of changing vellus hairs to terminal hairs.

In the case of male pattern alopecia, the main symptom is the change of terminal hairs to vellus hairs. Therefore, the composition for hair growth of the present invention (Tafluprost) is expected to be an effective preventive or treating agent for male pattern alopecia.

Further, the 15,15-difluoroprostaglandin F2α derivative is considered to be different in the hair growth action mechanism from conventional minoxidil, finasteride, etc., and therefore, when such medicines different in the hair growth action mechanism are used in combination, a higher hair growth effect can be expected.

Here, the "combination" means both a combined administration i.e. an administration wherein a hair growth agent containing as an active ingredient at least one 15,15-difluoroprostaglandin F2α derivative, and a hair growth agent having an action mechanism different therefrom (minoxidil, finasteride, etc.), are administered in the form of separate medicines, and a mixed medicine administration i.e. an administration in the form of a single medicine having such active ingredients formulated into one medicine.

The hair growth component which may be combined, includes minoxidil, finasteride, t-flavanone, adenosine, saitopurin, pentadecane, ketoconazole, etc. Among them, at least one member selected from the group consisting of minoxidil and finasteride is preferred, and from the viewpoint of the combined effects and promotion effects, minoxidil is most preferred.

The "15,15-difluoroprostaglandin F2α derivative" in the present invention means a compound represented by the following formula (1) or a salt thereof, among 15,15-difluoroprostaglandin F2α derivatives or salts thereof as disclosed in European Patent Application Publication No. 850926, JP-A-2004-002462, JP-A-10-259179, JP-A-2002-293771 and JP-A-2003-321442. 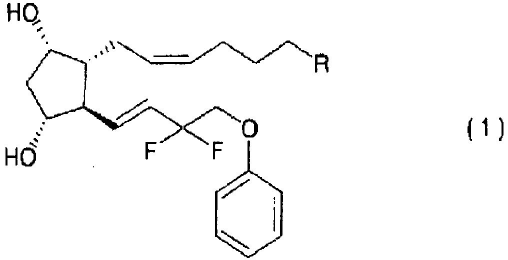 wherein R is a carboxy group or an alkoxycarbonyl group.

In the above formula (1), "alkoxy" represents C₁₋₆ linear or branched alkoxy. As specific examples, methoxy, ethoxy, n-propoxy, n-butoxy, n-pentyloxy, n-hexyloxy, isopropoxy, isobutoxy, sec-butoxy, tert-butoxy, isopentyloxy, etc. may be mentioned.

As a particularly preferred 15,15-difluoroprostaglandin F2α derivative, a compound of the above formula (1) wherein R is a carboxy group or a salt thereof, or an isopropoxycarbonyl group, may be mentioned, and the latter is known by a common name "Tafluprost".

Such a 15,15-difluoroprostaglandin F2α derivative can form a salt with e.g. an alkali metal such as lithium, sodium or potassium, an alkaline earth metal such as calcium or magnesium, or ammonia. Particularly, a salt with an alkali metal such as sodium or potassium is preferred.

Such a salt of the compound of the formula (1) is also useful as an active ingredient in the present invention.

The blend amount of the 15,15-difluoroprostaglandin F2α derivative in the composition for hair growth (as described later) varies depending upon the formulation, the application, the administration program, etc., but in any case, an effective amount thereof is blended. Namely, the dose of the active ingredient varies also depending upon the symptom, age, etc. of the person to be dosed, and therefore, the effective amount is determined by taking them into consideration in a comprehensive manner.

The "composition for hair growth" containing as an active ingredient the above compound or a salt thereof may be called as a "hair growth agent" and may be used as a medicine, a care product, a cosmetic product or the like for promoting the growth of hair, such as hair growth, hair restoration, hair increase, pilatory treatment, etc.

More specifically, it is useful as a preventive or treating agent for a disorder relating to hair such as alopecia and can be used also as a hair care product (a product for external application for scalp hair) such as a hair growth agent, a hair restoration agent, a hair increase agent, a pilatory treatment agent, etc.

The composition for hair growth of the present invention is preferably an agent for external application.

Here, the agent for external application is one to be applied to an outer skin and includes formulations such as liquid, cream, ointment, jelly, powdered medicine, etc.

A liquid agent for external application means an agent for external application in a liquid form.

The "hair" is not particularly limited so long as it is human or animal body hair, but scalp hair (hair on the head) is particularly preferred.

The composition for hair growth of the present invention is useful not only as a preventive or treating agent for e.g. a male pattern alopecia, but also as a hair care product for patients with a male pattern alopecia.

As a hair care product such as a hair growth agent, a hair restoration agent, a hair increase agent, a pilatory treatment agent, or the like, an agent for external application such as a hair agent, a hair lotion, a hair cream, a hair jelly, a hair shampoo, a hair conditioner or the like is preferred, and a liquid agent for external application such as a hair agent, a hair lotion or the like is particularly preferred.

When the composition for hair growth is used as a medicine, it may be externally or orally administered, but external administration is preferred. As such an external administration medicine form, a liquid agent, a cream, a jelly or a powdered medicine may, for example, be mentioned. A liquid agent or a cream is preferred, and a liquid agent for external application is particularly preferred.

In the following, a preferred embodiment of the liquid agent for external application will be described. Needless to say, however, the following exemplified blend components may be applied also to a composition for hair growth in a form other than the liquid agent for external application, although their preferred blend amounts may vary depending upon the formulation.

A liquid agent for external application may be formulated by using, as a base material, one or more polar solvents having a boiling point of at most 100°C. Such polar solvents are important in order to dissolve the 15,15-difluoroprostaglandin F2α derivative, and it is thereby possible to uniformly apply the agent. Further, they have effects to accelerate drying of the scalp and present a pleasant cooling sensation, and an antiseptic activity.

The polar solvents having a boiling point of at most 100°C may, for example, be ethanol, modified ethanol, water, isopropanol, etc., and they may be used in combination. Most preferred may be ethanol or a mixture of ethanol and water.

The blend amount of such polar solvents is preferably from 40 to 95 v/v%, particularly preferably from 50 to 80 v/v%, in the liquid agent for external application.

The blend amount of the 15,15-difluoroprostaglandin F2α derivative in the liquid agent for external application varies depending upon the product formulation (e.g. whether it is a medicine or a hair care product), the object to be applied, the application method, etc. The effective amount is preferably from 0.00001 to 0.01 w/v%, more preferably from 0.0001 to 0.001 w/v%.

The agent for external application may be applied from once to a few times per day.

In the case of a combined use with an active ingredient different in the hair growth working mechanism like minoxidil, finasteride or the like mentioned above (hereinafter referred to also as "another active ingredient"), the blend amount of such "another active ingredient" is preferably from 0.1 to 10 w/v%, more preferably from 0.5 to 5 w/v%, in the agent for external application.

Further, when the amount of the 15,15-difluoroprostaglandin F2α derivative is taken as 1, the amount of another active ingredient is preferably from 1:100 to 1:10,000, more preferably from 1:500 to 1:5,000, by mass ratio, in order to obtain hair growing effects by such a combination.

In a case where the viscosity as the agent for external application is deficient, it is possible to adjust the viscosity by blending a thickener such as ethyl cellulose, a carboxymethyl cellulose salt, a carboxy vinyl polymer, hydroxypropyl cellulose, hydroxypropylmethyl cellulose, xanthane gum, methyl cellulose, a fatty acid monoglyceride (such as pentadecanoic acid monoglyceride), a polyhydric alcohol (such as glycerin, propylene glycol or1,3-butylene glycol) or cetanol. As the thickener, ethyl cellulose, a carboxymethyl cellulose salt, a polyhydric alcohol or the like is preferred.

It is possible to reduce the surface tension of the agent for external application by blending a surfactant.

The surfactant may, for example, be a sorbitan fatty acid ester, a polyglycerin fatty acid ester, a polyoxyethylene sorbitan fatty acid ester, a polyoxyethylene sorbit fatty acid ester, a polyoxyethylene glycerin fatty acid ester, a polyethylene glycol fatty acid ester, a polyoxyethylene alkyl ether, a polyoxyethylene polyoxypropylene alkyl ether, a polyoxyethylene alkylphenyl ether, a polyoxyethylene hydrogenated castor oil, a polyoxyethylene castor oil, a polyoxyethylene beeswax derivative, a polyoxyethylene lanolin derivative, a polyoxyethylene alkylamide, a polyoxyethylene alkylamine, a lecithin derivative or a polymer emulsifier. Two or more of them may be used in combination.

By blending an organic solvent having a boiling point of at least 150°C in combination, the application may be carried out more uniformly at the time of applying the agent for external application. The organic solvent having a boiling point of at least 150°C may, for example, be ethylene glycol, diethylene glycol, triethylene glycol, 1,3-butylene glycol, dipropylene glycol, glycerin, propylene glycol or polyethylene glycol. As one preferred from the viewpoint of hair growing effects, ethylene glycol, diethylene glycol, triethylene glycol, propylene glycol, dipropylene glycol or the like may be mentioned. Two or more of them may be used in combination.

To the agent for external application, additives which are commonly used in this technical field may be blended as the case requires, such as a dissolution assistant (such as various plant oils, various animal oils, an alkyl glyceryl ether, lactic acid, phosphoric acid or sodium hydroxide), a hydrocarbon (such as liquid paraffin or squalane), an antioxidant (such as dibutylhydroxytoluene, sodium pyrosulfite, tocopherol, sodium edetate, ascorbic acid or isopropyl gallate), an emulsion stabilizer, a gelling agent (such as a polymer), an adhesive, a refreshing agent (such as menthol, peppermint or camphor) and a vitamin (such as vitamin E or vitamin A).

Further, as other components, commonly used components may be blended, such as a vasodilator as a component commonly used for a hair growth agent (such as carpronium chloride, benzyl nicotinate, swertia Japonica extract, Panax ginseng extract, vitamin E acetate or Capsicum Tincture), an antihistamine (such as diphenhydramine hydrochloride or isothipendyl hydrochloride), an anti-inflammatory agent (such as glycyrrhetinic acid or guaiazulene), a keratolytic medicine (such as urea or salicylic acid), a bactericidal agent (such as chlorhexidine gluconate, isopropyl methyl phenol, a quaternary ammonium salt, Hinokitiol or Piroctone olamine), a moisturizer (such as sodium hyaluronate or chondroitin sulfate), extracts from various plants or animals (such as Taxus cuspidate, moutan bark, Glycyrrhiza, Hypericum erectum, aconiti tuber, loquat, Artemisia capillaris, comfrey, Angelica keiskei, saffron, Gardenia Fruit, rosemary, Common Sage, Saussurea costus, Chinese Materia Medica, Humulus lupulus or Placenta), a vitamin (such as retinol acetate, pyridoxine hydrochloride, ascorbic acid, thiamine nitrate, cyanocobalamin or biotin), a fragrance, and a pigment.

The above-mentioned optional components may be suitably used also for compositions for hair growth other than the agent for external application.

Particularly, not only to the liquid agent but also to a hair care product other than the liquid agent, a thickener, a surfactant, an organic solvent having a boiling point of at least 150°C, etc. may be blended. Further, as the case requires, components which are commonly used for cosmetic products for hair growth, may be blended, such as water, a dissolution assistant, a hydrocarbon, an antioxidant, an emulsion stabilizer, a gelling agent, an adhesive, a refreshing agent, a vitamin, a fragrance, a pigment, etc.

A cosmetic method for treating hair according to the present invention is a non-therapeutic cosmetic method which includes application of the above composition for hair growth to hair. It is particularly preferably employed for treatment of scalp hair.

Here, the cosmetic method is a method to keep hair healthy and to make the appearance of the surface beautiful, and by using the composition for hair growth of the present invention, it is possible to carry out hair growth, pilatory treatment, etc. thereby to make the hair beautiful without bringing about blackening of the skin.

### EXAMPLES

Now, Preparation Example and pharmacological test results of the composition (medicine) for hair growth of the present invention will be described.

It should be understood that these exemplifications are intended for better understanding of the present invention and by no means limit the scope of the present invention.

### 1. Preparation of medicine

1.5 mg of Tafluprost was dissolved in 100 mL of a 75 v/v% ethanol aqueous solution to prepare 0.0015% Tafluprost (a 75 v/v% ethanol aqueous solution of 0.0015 w/v% Tafluprost).

For the purpose of comparison, a 50 v/v% ethanol aqueous solution of 1 w/v% minoxidil (1% minoxidil) was prepared.

### 2. Pharmacological test

### (1) Test method

Hairs on the back of each C3H/HeNSIc mouse (male, 8 weeks old) were carefully dehaired in an area of 2cm × 3cm to form a medicine application surface. The next day (the day before initiation of the test), mice which were confirmed to have no damage on the application surface of the back, to have a pink color over the entire application surface and to be in the telogen stage of hair growth, were bred in a stainless steel cage (185W × 260D × 175H (mm)) in three groups each group consisting of 10 mice.

On the application surface of mice, (a) distilled water, (b) the above 0.0015% Tafluprost, or (c) the above 1% minoxidil was applied in an amount of 150 µL once every day for 28 days to the respective groups, while being careful not to have the surface stimulated by a finger.

### (2) Evaluation method

Every day, the application surface was inspected before the application, and the day on which hair growth was observed, was taken as the hair growth day. The hair growth score was evaluated in accordance with the following evaluation standards, based on the area where hair growth was observed on the skin surface.

**[Table 1] Evaluation standards for hair growth score**

| Score | Evaluation |
|---|---|
| 0 | No hair growth observed |
| 1 | Percentage of hair growth area being from 0 to less than 10% |
| 2 | Percentage of hair growth area being from 10 to less than 20% |
| 3 | Percentage of hair growth area being from 20 to less than 30% |
| 4 | Percentage of hair growth area being from 30 to less than 40% |
| 5 | Percentage of hair growth area being from 40 to less than 50% |
| 6 | Percentage of hair growth area being from 50 to less than 60% |
| 7 | Percentage of hair growth area being from 60 to less than 70% |
| 8 | Percentage of hair growth area being from 70 to less than 80% |
| 9 | Percentage of hair growth area being from 80 to less than 90% |
| 10 | Percentage of hair growth area being from 90 to 100% |

### 3. Results

**[Table 2]**

| | Hair growth day | Hair growth score | |
|---|---|---|---|
| Application material | Average number of days ± standard error (day) | 10th day | 15th day |
| Distilled water | 12.6±0.5 | 0.2±0.2 | 4.3±1.0 |
| 0.0015% Tafluprost | 10.0*±0.0 | 1.7*^{a}±0.2 | 9.4*^{a}±0.3 |
| 1% minoxidil | 10.4*±0.4 | 2.0*^{b}±0.4 | 9.1*^{b}±0.9 |

| | | | |
|---|---|---|---|
| * Significant difference to the group to which distilled water was applied (p<0.05). a Hair growth without blackening of the skin as visually observed. b Hair growth with blackening of the skin as visually observed. | | | |

In the Tafluprost-applied group to which 0.0015% Tafluprost was applied, in each of evaluation items for the hair growth day and the hair growth score, significant hair growth was observed as compared to the distilled water-applied group, and the results were equal to the 1% minoxidil-applied group as the positive control.

Hair growth in the Tafluprost-applied group was different from usual hair growth observed in the minoxidil-applied group and was specific without blackening of the skin as visually observed, whereby the difference from minoxidil in the hair growth working mechanism was apparent.

### INDUSTRIAL APPLICABILITY

The composition for hair growth of the present invention is useful as a preventive or treating agent for a disorder relating to hair such as alopecia, without blackening of the skin or tanning of the skin; and is useful also as a hair care product for the purpose of hair growth, hair restoration, hair increase, pilatory treatment, etc.

The entire disclosure of Japanese Patent Application No. 2012-123316 filed on May 30, 2012 including specification, claims and summary is incorporated herein by reference in its entirety.

## Claims

1. A composition for hair growth, which comprises as an active ingredient at least one member selected from the group consisting of a compound represented by the formula (1) and a salt thereof: 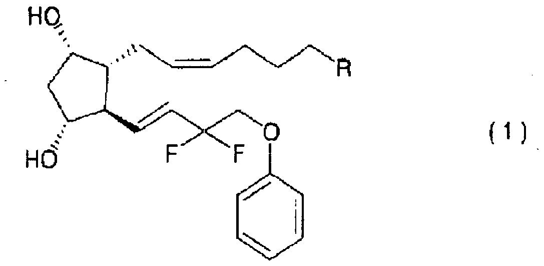 wherein R is a carboxy group or an alkoxycarbonyl group.

2. The composition for hair growth according to Claim 1, wherein in the compound represented by the formula (1), R is an isopropyloxycarbonyl group.

3. The composition for hair growth according to Claim 1 or 2, which further contains as an active ingredient at least one member selected from the group consisting of minoxidil and finasteride.

4. The composition for hair growth according to any one of Claims 1 to 3, which is used for prevention or treatment of male pattern alopecia.

5. The composition for hair growth according to any one of Claims 1 to 4, which is a liquid for external use comprising from 0.00001 to 0.01 w/v% of at least one member selected from the group consisting of a compound represented by the above formula (1) and a salt thereof, and from 40 to 95 v/v% of ethanol.

6. The composition for hair growth according to Claim 5, wherein the liquid for external use contains a polar solvent having a boiling point of at most 100°C.

7. The composition for hair growth according to Claim 6, wherein the polar solvent is ethanol, or a mixture of ethanol and water.

8. The composition for hair growth according to any one of Claims 5 to 7, which is a hair care product.

9. A cosmetic method for hair, which comprises applying to hair the composition for hair growth as defined in any one of Claims 1 to 8.
